# EUROPEAN PATENT APPLICATION

(11) **EP 4 671 360 A1**
(43) Date of publication of application: **31.12.2025**
(21) Application number: 24759817.0
(22) Date of filing: 22.02.2024
(51) Int. Cl.: C12N 1/20, A61P 1/16, A61P 3/04, A61P 3/10, A61P 25/22, A61P 25/24

(54) **STRAIN OF BIFIDOBACTERIUM LONGUM AND USE THEREOF**

(30) Priority: 22.02.2023 ES 202330135
(71) Applicant: Agencia Estatal Consejo Superior de Investigaciones Científicas (CSIC), 28006 Madrid (ES)
(72) Inventor: SANZ HERRANZ, Yolanda, Madrid 28006 (ES); AGUSTÍ FELIU, Ana, 28006 Madrid (ES); ROSSINI, Valerio, 28006 Madrid (ES); TOLOSA ENGUIS, Verónica, 28006 Madrid (ES); FRANCÉS CUESTA, Carlos, 28006 Madrid (ES); FLOR DURO, Alejandra, 28006 Madrid (ES); MOLINA MENDOZA, Gara Verónica, 28006 Madrid (ES)
(74) Representative: Pons IP
(86) International application number: PCT/ES2024/070105
(87) International publication number: WO 2024/175823

(57) **Abstract**

The invention relates to the B. *longum* strain CECT 30763, to its cellular components, metabolites, and secreted molecules, and to compositions comprising the above-mentioned products, as well as to uses thereof in the prevention or treatment of mood disorders, such as depression and anxiety, cardiometabolic disorders or liver disorders, alone or presenting as comorbidities.

## Description

The present invention falls within the field of therapeutic activity of pharmaceutical compositions or preparations, as well as in the field of foods. In particular, the present invention relates to *Bifidobacterium longum* strain CECT 30763 and to the use thereof for the prevention or treatment of mood disorders, such as depression and anxiety, cardiometabolic disorders or liver disorders, alone or presenting as comorbidities.

### STATE OF THE ART

Mental disorders, such as depression and anxiety, and cardiometabolic disorders, such as cardiovascular diseases, liver fibrosis and type 2 diabetes, are among the leading causes of disability, morbidity and mortality worldwide. These diseases have a high rate of comorbidity, i.e. they present simultaneously. Depression and anxiety present together in around 50% of cases. These psychiatric pathologies also increase the risk of cardiometabolic disorders (such as metabolic syndrome), diabetes and cardiovascular diseases) and liver disorders, and vice versa, so they often present simultaneously. The existence of comorbidities, in addition to making diagnosis difficult, also worsens the prognosis of the disease and the response to therapies.

Mood disorders and, particularly, depression and anxiety, are highly prevalent and a leading cause of disability worldwide. The total cost of mental disorders is estimated at 798 billion, of which mood disorders represent an annual direct and indirect cost of around 118 billion. The term depression includes major or unipolar depression, typical or melancholic and atypical depression, prenatal and postpartum depression, bipolar disorder, psychotic depression, dysthymia, depressive personality disorder, seasonal affective disorder, and mood disorder induced by substance abuse or drug use. Anxiety disorders include panic disorder, generalised anxiety disorder, social phobia and specific phobia, agoraphobia, and anxiety due to a general medical condition. The efficacy of current therapies for these conditions is quite limited. It is estimated, for example, that only about 50% of treatments with antidepressants are fully effective; many patients are left with sub-clinical symptomatology and often relapse, and a subgroup of patients do not improve at all.

Depression shows a high comorbidity with mental disorders (for example, anxiety) and physical disorders (for example, cardiometabolic disorders such as metabolic syndrome, diabetes, and cardiovascular disease), which worsen the course of the disease, reduce therapeutic response, and increase the risk of developing it. Anxiety also has a high comorbidity with mental and cardiometabolic diseases, such as high blood pressure and coronary heart disease. In addition, it increases the risk of suffering major adverse coronary events, such as myocardial infarction, left ventricular failure, coronary revascularisation procedures and stroke, which occur in people with cardiovascular diseases.

Stress is one of the main risk factors for the development of depression and anxiety. Likewise, stress has been associated with the development of cardiovascular disease, metabolic syndrome, type 2 diabetes mellitus and obesity, as well as liver disorders, inducing liver fibrosis, for example. In the liver, stress can reduce hepatic blood flow, cause tissue damage, increase transaminase levels, promote the development of certain liver diseases and the onset of liver fibrosis associated with increased inflammation. Inflammation in the liver caused by the activation of pathogen-associated molecular pattern (PAMPS) or damage-associated molecular pattern (DAMPS) recognition receptors induces the activation and transformation of quiescent hepatic stellate cells into myofibroblasts, which are highly fibrogenic. Liver fibrosis is a disease characterised by the excessive accumulation of extracellular matrix proteins in the liver, primarily collagen, which hinders its proper function and furthermore promotes the development of hepatocellular carcinoma.

The high comorbidity of mental and cardiometabolic pathologies suggests that they may be triggered by the same risk factors and share pathophysiological mechanisms. This also suggests that there may be common solutions to prevent or treat these comorbidities. Chronic inflammation and altered stress response are among the pathophysiological mechanisms linking depression and anxiety to cardiometabolic diseases. Chronic inflammation and dysregulation of the stress response mediated by the hypothalamic-pituitary-adrenal (HPA) axis may result from altered communication between the gut and the brain (gut-brain axis). This axis is largely regulated by the gut microbiota, so modulation thereof represents a preventive and therapeutic target. In fact, it can be seen in animal models that an increase in the stress-induced HPA axis response causes alterations in the gut microbiota; in turn, the altered microbiota contributes to behavioural and mood disorders. Animal studies also show that the specific configuration of the gut microbiota influences stress response, aggravating or improving its neurochemical and behavioural consequences through mechanisms that coordinate the dialogue between the immune, endocrine and nervous systems.

The use of gut bacteria for the treatment of conditions such as depression, anxiety, cardiometabolic or liver disorders has been investigated, considering these conditions as individual and independent entities. Given the high prevalence and clinical importance of these conditions, the state of the art highlights the need for alternative bacterial strains for the prevention and/or treatment thereof, especially considering the lack of studies on the use and effectiveness of microbial strains for these conditions when presented as comorbidities, which make their management, including prevention and treatment, much more complex.

### DESCRIPTION OF THE INVENTION

The present invention relates to a *Bifidobacterium longum (B. Longum)* strain, specifically, a *B. longum* strain deposit number CECT 30763, and derived strains, to cellular components, metabolites, secreted molecules of said strain, and to compositions comprising the above-mentioned products, as well as to the use for the prevention and/or treatment of mood disorders, such as depression and anxiety; cardiometabolic disorders, such as obesity, metabolic syndrome, type 2 diabetes, and cardiovascular diseases; or liver disorders, such as hepatic steatosis and liver fibrosis. Since many of these disorders have a high rate of comorbidity, the invention also relates to the *B*. *longum* strain CECT 30763 for use in the prevention and/or treatment of psychiatric, cardiometabolic and/or liver comorbidities.

The inventors have identified and isolated this *B*. *longum* strain CECT 30763 from faeces of healthy volunteers and have shown that the strain has the abilityto exert beneficial effects on depressive and anxiogenic behaviour, as well as on cardiometabolic and liver disorders in animals in which these comorbidities are induced by exposure to chronic social stress.

In particular, the inventors have demonstrated that oral administration of *B*. *longum* CECT 30763 to the animal model of social stress attenuates depressive and anxiogenic behaviour, as shown by the results of the behavioural tests described in Example 2.

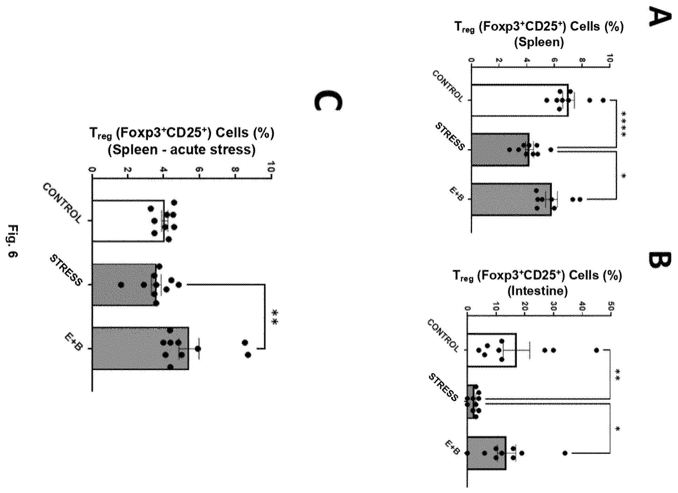

Moreover, the strain of the invention reduced plasma levels of chemokines and cytokines, specifically, CXCL9, CXCL10, CCL2, IL-6 and TNF-α, the increase in which is associated with depression and anxiety in said animal model of stress and in humans, restoring them until reaching levels similar to those of the control model (Example 5). These chemokines and cytokines are also inflammatory mediators that are also increased in cardiometabolic pathologies, so results indicate that the strain of the invention exerts a positive role in disorders of this type. In this sense, the anti-inflammatory effect of *B*. *longum* strain CECT 30763 may also derive from the increase in regulatory T-lymphocytes (Tregs), which are associated with an anti-inflammatory role, and the levels of which were significantly decreased in both the spleen and the intestine as a consequence of chronic stress, with the strain being able to increase them (Figures 6A and 6B). Furthermore, after the first exposure to stress (acute stress), the strain of the invention induced a significant increase in Tregs, indicating that this anti-inflammatory and regulatory effect is exerted at early stages and contributes to preventing the adverse effects of chronic stress (Figure 6C).

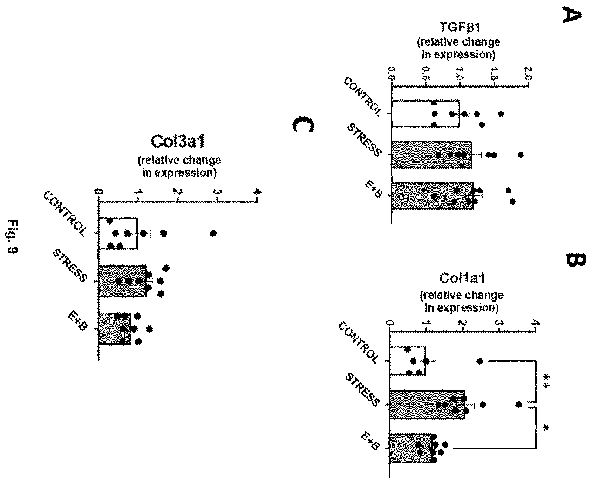

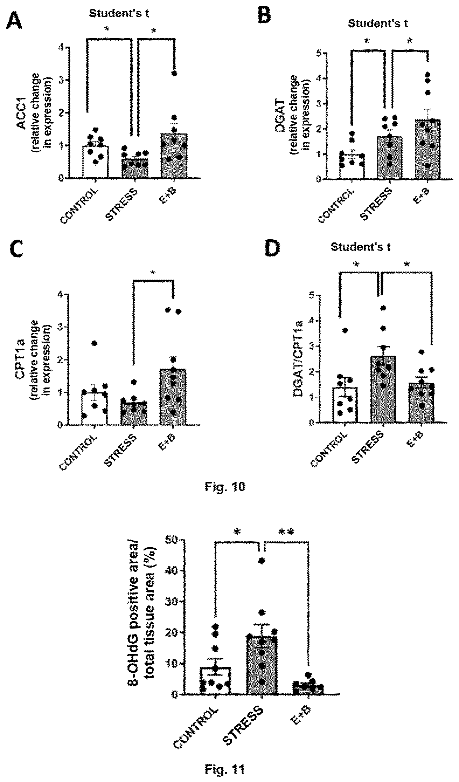

Other effects exerted by *B. longum* strain CECT 30763 include improved endocrine and dopamine responses, as it reduces the overproduction of corticosterone and elevated dopamine levels induced by chronic stress (see Example 4). B. *longum* strain CECT 30763 also exerts beneficial effects on the liver (see Example 5), modifying the expression of enzymes related to fatty acid metabolism in the liver altered by stress, as well as changes in the expression of profibrotic molecules (Figures 9A, 9B and 9C). B. *longum* strain CECT 30763 also exerts beneficial effects on the heart by reducing oxidative stress (Example 6, Figure 11).

### Strain of the invention and derivatives

**In** one aspect, the present invention relates to a *Bifidobacterium longum* strain with deposit number CECT 30763, hereinafter "strain of the invention" or *"B. longum* CECT 30763".

*B. longum* strain CECT 30763 (G121) was isolated from faeces of healthy humans and deposited on 13 December 2022 under the Budapest Treaty in the Spanish Type Culture Collection as the International Depositary Authority (Edificio 3 CUE, Parc Científic Universitat de Valencia, Catedrático Agustín Escardino, 9, 46980, Paterna (Valencia) SPAIN). The assigned deposit number was CECT 30763.

The scientific classification of the strain of the invention is: Domain: *Bacterium;* Phylum:
*Actinobacteria;* Class: *Actinobacteria;* Order: *Bifidobacteria;* Family: *Bifidobacteriaceae;* Genus: *Bifidobacterium;* Species: *B. longum.*

Members of the genus *Bifidobacterium* are among the microorganisms that colonise the human gastrointestinal tract and are believed to confer health benefits on their host. Bifidobacteria have been commercially exploited as probiotic agents due to said health benefits and because they are generally recognised as safe.

Bifidobacteria are gram-positive, non-motile, and often branched anaerobic bacteria. They are one of the main genera of bacteria that make up the mammalian gut microbiota and comprise more than 30 different species. Among such bacteria, B. *longum* is a catalase-negative bacterium with a branched rod shape.

Another aspect of the present invention relates to a strain derived from *B*. *longum* strain CECT 30763, wherein said strain maintains or improves the capabilities of the original strain, which are described throughout the present invention.

In a preferred embodiment, alone or in combination with other preferred embodiments, the strain derived from *B*. *longum* CECT 30763 has a sequence identity of at least 90%, 91%, 92%, 93%, 94%, 95%, 96%, 97%, 98%, 99% with the sequence SEQ ID NO: 5. The sequence SEQ ID NO: 5 corresponds to the 16S-rRNA gene sequence of *B*. *longum* strain CECT 30763.

In the present invention, "sequence identity" is understood as the degree of similarity between two nucleotide sequences obtained by aligning the two sequences. Depending on the number of common residues between the aligned sequences, a degree of identity expressed as a percentage is obtained. The degree of identity between two nucleotide sequences can be determined by conventional methods, for example, using standard sequence alignment algorithms known in the state of the art, such as BLAST [Altschul S.F. et al. Basic local alignment search tool. J Mol Biol. 1990 Oct 5; 215(3):403-10]. The BLAST programs, for example, BLASTN, BLASTX, and TBLASTX, and TBLASTN, are in the public domain on the *The National Center for Biotechnology Information* (NCBI) website.

The derived microorganism may be produced naturally (for example, spontaneous mutants) or intentionally, by mutagenesis methods known in the state of the art such as, but not limited to, the growth of the original microorganism in the presence of mutagenic or stress-inducing agents, or by genetic engineering aimed at modifying specific genes. Therefore, in a preferred embodiment, the strain of the invention or the strain derived from the species *B*. *longum* is a genetically modified mutant or a spontaneous mutant. The terms "mutant strain" or "derived strain" can be used interchangeably.

The B. *longum* strain CECT 30763 or any mutant or derivative thereof can be used in any way that exerts the described effects, such as, for example, according to a preferred embodiment of the present invention, the *B*. *longum* strain is in the form of viable cells (culturable or non-culturable), or according to another preferred embodiment of the invention, *B. longum* strain CECT 30763 is in the form of non-viable cells ("dead" cells inactivated by any technique known in the state of the art such as, but not limited to, heat, freezing or ultraviolet radiation). Therefore, in a more preferred embodiment, non-viable cells of *B*. *longum* strain CECT 30763 are selected from the list consisting of: heat-treated, frozen, freeze-dried or treated by ultraviolet radiation.

As used herein, the term "non-viable" (or "inanimate", "inactive" or "inactivated", which terms may be used interchangeably herein) refers to any metabolically or physiologically inactive microorganism, i.e., it does not retain its metabolic activity and elongation capacity after the administration of nutrients. Viability is independent of the ability of the microorganism to form colonies on solid media.

Another aspect of the present invention relates to cellular components, metabolites, secreted molecules or any of the combinations thereof, obtained from the strain of the invention, the derived strain, or from a combination of microorganisms comprising at least one strain of the invention and/or the derived strain.

Among the cellular components of the bacterium, the components of the cell wall could be included (such as, for example, but not limited to, peptidoglycan), as well as nucleic acids, components of the membrane, or others, such as proteins, lipids and carbohydrates and the combinations thereof, such as lipoproteins, glycolipids or glycoproteins. Metabolites include any molecule produced or modified by the bacterium as a consequence of the metabolic activity thereof during its growth, the use thereof in technological processes (for example, but not limited to, food or drug manufacturing processes), during product storage or during gastrointestinal transit. Examples of these metabolites are, but not limited to, organic and inorganic acids, proteins, peptides, amino acids, enzymes, lipids, carbohydrates, lipoproteins, glycolipids, glycoproteins, vitamins, salts, metals or nucleic acids. Secreted molecules include any molecule exported or released to the outside by the bacterium during its growth, the use thereof in technological processes (for example, food or drug manufacturing), product storage or gastrointestinal transit. Examples of these molecules are, but not limited to, organic and inorganic acids, proteins, peptides, amino acids, enzymes, lipids, carbohydrates, lipoproteins, glycolipids, glycoproteins, vitamins, salts, metals or nucleic acids.

### Compositions of the invention and uses

Another aspect of the present invention relates to a composition, hereinafter "composition of the invention", comprising the strain of invention, the derived strain and/or cellular components, metabolites, secreted molecules of the strain of the invention or the derived strain, or any of the combinations thereof.

The composition, defined in a general way, is a set of components that is made up of at least the strain of the invention and/or the derived strain at any concentration; or at least cellular components, metabolites, secreted molecules of the strain of the invention or the derived strain, or any of the combinations thereof; or a combination thereof.

**In** a preferred embodiment, the composition of the invention has a concentration of the strain of the invention and/or the derived strain of between 10⁴ and 10¹⁴ colony-forming units (cfu) per gram or millilitre of final composition.

**In** another preferred embodiment, the composition of the invention may further comprise at least an additional microorganism other than the strain of the invention or the derived strain, and/or its cellular components, metabolites or secreted molecules, or any combination thereof. For example, but without limitation, the additional microorganism that can be part of said composition is selected from at least one of the following groups:
- at least one lactic bacterium or bifidobacterium of intestinal, food or environmental origin. The lactic bacterium is selected from the list comprising, without limitation, a bacterium of the genus *Bifidobacterium, Lactobacillus, Lactococcus, Enterococcus, Propionibacterium, Leuconostoc, Weissella, Pediococcus* or *Streptococcus;*
- at least one strain of other phylogenetic groups, genera or species of prokaryotes of intestinal, food or environmental origin, such as, but not limited to, Archaea, Firmicutes, Bacteroidetes, Proteobacteria, Actinobacteria, Verrucomicrobia, Fusobacteria, Methanobacteria, Spirochaetes, *Fibrobacteres,* Deferribacteres, *Deinococcus, Thermus, Cyanobacteria, Methanobrevibacterium, Peptostreptococcus, Ruminococcus, Coprococcus, Subdolingranulum, Dorea, Bulleidia, Anaerofustis, Gemella, Roseburia, Catenibacterium, Dialister, Anaerotruncus, Staphylococcus, Micrococcus, Propionibacterium, Enterobacteriaceae, Faecalibacterium, Bacteroides, Parabacteroides, Prevotella, Eubacterium, Akkermansia, Bacillus, Butyrivibrio or Clostridium;*
- at least one strain of fungus or yeast such as, but not limited to, one belonging to the genus *Saccharomyces, Candida, Pichia, Debaryomyces, Torulopsis, Aspergillus, Rhizopus, Mucor* or *Penicillium.*

Said additional microorganism can be a strain of the same species or of a different species or a taxonomic group of microorganisms from the one corresponding to the strain of the invention. The cells comprising the composition may be non-viable or viable and be in any phase of the developmental or growth state (latent, exponential, stationary, etc.), regardless of the morphology it has. In a particular embodiment, said additional microorganism comprises at least one intestinal bacterium or one lactic bacterium.

Optionally, in another particular embodiment, the composition of the invention may further comprise at least one bioactive component (active substance, active ingredient or therapeutic agent), such as food components, plant products and/or drugs. The term "bioactive component" refers to a compound with biological activity within the scope of application of the patent which can improve or complement the activity of B. *longum* strain CECT 30763, including food ingredients or components (for example and without limitation: polyunsaturated fatty acids, conjugated linoleic acid, prebiotics, fibre, guar gum, glucomannan, chitosan, copper picolinate, calcium, etc.), other probiotics, plants, plant extracts or components and drugs.

Furthermore, the composition of the invention may be formulated for pharmaceutical administration, that is, forming part of pharmaceutical products to be administered to the subject by any means of administration. Therefore, in a particular embodiment, the composition of the invention is a pharmaceutical composition, hereinafter also referred to as "pharmaceutical composition of the invention". The pharmaceutical composition is a set of components that is made up of at least the strain of the invention and/or the derived strain at any concentration, or at least cellular components, metabolites, secreted molecules of the strain of the invention or the derived strain, or any of the combinations thereof, having at least one application in improving the physical or physiological or psychological well-being of a subject, which implies an improvement in the general state of their health or a reduction in the risk of disease. Said pharmaceutical composition can be a medicament, hereinafter also referred to as the medicament of the invention.

Therefore, in another aspect, the invention relates to the strain of the invention, a strain derived from it, a cellular component, metabolite, secreted molecule or any of the combinations thereof, obtained from the strain of the invention or the derived strain, or the composition of the invention, for use as a medicament.

The term "medicament" has a more limited meaning than the meaning of "pharmaceutical composition", as defined in the present invention, since medicament necessarily implies a preventive or therapeutic effect. The medicament to which the present invention relates may be for human or veterinary use. The "medicament for human use" is any substance or combination of substances that has properties for treating or preventing diseases in humans or that can be used in humans or administered to humans for the purpose of restoring, correcting, or modifying physiological functions by exerting a pharmacological, immune or metabolic effect, or establishing a medical diagnosis. The "medicament for veterinary use" is any substance or combination of substances that has curative or preventive properties with respect to animal diseases or that can be administered to an animal for the purpose of restoring, correcting, or modifying their physiological functions by exerting a pharmacological, immune or metabolic effect, or establishing a veterinary diagnosis. "Veterinary medicaments" will also be considered "premixes for medicated feed" prepared to be incorporated into a feed.

In addition to the requirement of therapeutic efficacy wherein said pharmaceutical composition may require the use of other therapeutic agents, there may be additional fundamental reasons which oblige or recommend to a great extent the use of a combination of a compound of the invention and a bioactive component, wherein said bioactive component is attributed with appropriate activity in order to constitute a medicament. Said compound of the invention obviously refers to the strain of the invention, or the strain derived from it, or the cellular components, metabolites, secreted molecules or any of the combinations thereof, obtained from the strain of the invention or the derived strain.

In a particular embodiment, the pharmaceutical composition of the invention further comprises at least one pharmaceutically acceptable vehicle and/or excipient.

The "carrier" is preferably an inert substance. The function of the carrier is to facilitate the incorporation of other compounds, allow better dosing and administration or give the pharmaceutical composition consistency and shape. Therefore, the carrier is a substance that is used in the medicament to dilute any of the components of the pharmaceutical composition of the present invention to a certain volume or weight; or that, even without diluting said components, is capable of allowing better dosing and administration or giving the medicament consistency and shape. When the form of presentation is liquid, the pharmaceutically acceptable carrier is the diluent.

The term "excipient" refers to a substance that aids the absorption of any of the components of the composition of the present invention, stabilises said components or aids the preparation of the pharmaceutical composition in the sense of giving it consistency or providing flavours that make it more pleasant. Thus, excipients could have the function of holding components together, such as starches, sugars, or celluloses, the function of sweetening, the function of colouring, the function of protecting the medicament, for example to isolate it from air and/or moisture, the function of filling a tablet, capsule or any other form of presentation, such as, for example, dibasic calcium phosphate, the function of disintegrating in order to facilitate the dissolution of components and the absorption thereof in the intestine, without excluding other types of excipients not mentioned herein. Therefore, the term "excipient" is defined as a material that, included in galenic forms, is added to active ingredients or to the associations thereof to allow for the preparation and stability thereof, to modify the organoleptic properties thereof or to determine the physicochemical properties of the pharmaceutical composition and the bioavailability thereof. The "pharmaceutically acceptable" excipient must allow for the activity of the compounds of the pharmaceutical composition, that is, it is compatible with said components.

Furthermore, as understood by a person skilled in the art, the excipient and the carrier must be pharmacologically acceptable, that is, the excipient and the carrier are allowed and evaluated so that no harm is caused to the organisms to which it is administered.

The pharmaceutical composition or medicament can be presented in any clinically acceptable route of administration and in a therapeutically effective amount. For example, it may be in a form adapted for oral, sublingual, nasal, intrathecal, bronchial, lymphatic, rectal, transdermal, inhaled or parenteral administration, preferably in a form adapted for oral administration. The pharmaceutical composition of the invention can be formulated in solid, semi-solid, liquid or gaseous forms, such as a tablet, capsule, powder, granule, ointment, solution, suppository, injection, inhalant, gel, microsphere or aerosol. In a preferred embodiment, alone or in combination with the other preferred embodiments, the pharmaceutical composition of the invention, or the medicament of the invention, is formulated in liquid or solid form.

In a preferred embodiment, alone or in combination with the other preferred embodiments, the composition of the invention, preferably the pharmaceutical composition of the invention or the medicament, is in a form adapted for oral, sublingual, nasal, intrathecal, bronchial, lymphatic, rectal, transdermal, inhaled or parenteral administration. In a more preferred embodiment, alone or in combination with the other preferred embodiments, the pharmaceutical composition of the invention or the medicament of the invention, is in a form adapted for oral administration.

The form adapted for oral administration refers to a physical state that can allow for the oral administration thereof. Examples of forms adapted for oral administration are, but not limited to, drops, syrup, herbal tea, elixir, suspension, extemporaneous suspension, drinkable vial, tablet, capsule, granulate, sachet, caplet, pellet, pill, lozenge or lyophilised form.

**In** an even more preferred embodiment, the pharmaceutical composition of the invention, or the medicament of the invention, is in a form adapted for oral administration selected from the list consisting of: drops, syrup, herbal tea, elixir, suspension, extemporaneous suspension, drinkable vial, tablet, capsule, granulate, sachet, caplet, pellet, pill, lozenge and lyophilised form.

The "galenic form" or "pharmaceutical form" is the disposition to which the active ingredients and excipients are adapted in order to constitute a medicament. It is defined by the combination of the form in which the pharmaceutical composition is presented by the manufacturer and the form in which it is administered.

**In** the present invention, the term "therapeutically effective amount" refers to the amount of the component of the pharmaceutical composition that when administered to a mammal, preferably a human, is sufficient to produce prevention and/or treatment, as defined below, of a disease or pathological condition of interest in the mammal, preferably a human. The therapeutically effective amount will vary, for example, according to the activity of the strain of the invention; the cellular components, metabolites, secreted molecules or any of the combinations thereof, in any form of presentation; the therapeutically effective amount will also vary according to the metabolic stability and duration of action of the compound; age, body weight, general state of health, sex and diet of the patient; the mode and time of administration; the excretion rate, the combination of drugs; the seriousness of the particular disorder or pathological condition; and the patient being subjected to therapy, but this can be determined by one skilled in the art according to his or her own knowledge and that description.

Alternatively to the pharmaceutical composition, the composition of the invention can also be a nutritional composition, also referred to herein as the "nutritional composition of the invention". Therefore, in another particular embodiment, the composition of the invention is a nutritional composition.

The term "nutritional composition" of the present invention refers to a food that, regardless of providing nutrients to the subject who takes it, beneficially affects one or more functions of the body, in a way that provides a better state of health and well-being. Consequently, said nutritional composition can be intended for the prevention and/or treatment of a disease or of the factor causing a disease. Therefore, the term "nutritional composition" of the present invention can be used synonymously with functional food or food for specific nutritional purposes or medicinal food.

**In** a preferred embodiment, the nutritional composition is a food, a supplement, a nutraceutical, a probiotic, a symbiotic, a postbiotic, a parabiotic or a psychobiotic.

**In** a more preferred embodiment, the food is selected from the list consisting of a dairy product, plant product, meat product, a snack, chocolate, beverage or baby food.

Examples of dairy products include, but are not limited to, a product derived from fermented milk (for example, but not limited to yoghurt or cheese) or non-fermented milk (for example, but not limited to, ice cream, butter, margarine, whey). In a more preferred embodiment, the food is a dairy product selected from the list consisting of yoghurt, cheese, ice cream, butter, margarine, whey, and any other product derived from fermented or non-fermented milk.

Examples of plant products include, but are not limited to, a grain in any form of presentation, fermented or non-fermented. In another more preferred embodiment, the food is a plant product, wherein the plant product is a fermented or non-fermented grain.

Examples of a beverage include, but are not limited to, any fruit or vegetable juice or non-fermented milk. **In** another more preferred embodiment, the food is a beverage, wherein said beverage is a fruit or vegetable juice or non-fermented milk.

The term "supplement", synonymous with any of the terms "dietary supplement", "nutritional supplement", or "food supplement", is a "food ingredient" intended to supplement food. Some examples of supplements are, but not limited to, vitamins, minerals, botanicals, amino acids and food components such as enzymes and glandular extracts. They are not presented as substitutes for conventional food or as a sole component of a meal or of the food diet but rather as a complement to the diet. Furthermore, the supplement can be presented in single or combined form and marketed in the form of, but not limited to, capsules, caplets, pellets and other similar forms, powder sachets, liquid ampoules and drop dispensers, and other similar forms of liquids and powders designed to be taken in a single quantity.

The term "nutraceutical" as used in the present invention refers to substances isolated from a food and used in a dosage form that have a beneficial effect on health.

The term "probiotic" as used in the present invention refers to live microorganisms that when administered in adequate amounts promote health benefits to the host organism.

The term "symbiotic" as used in the present invention refers to foods that contain a mixture of prebiotics and probiotics. As a general rule, they contain a prebiotic component that favours growth and/or metabolic activity and ultimately the effect of the probiotic with which it is combined, for example and without limitation, it may be the association of fructooligosaccharides or galactooligosaccharides with bifidobacteria.

The term "postbiotic" as used in the present invention refers to those foods or supplements which contain the substances or metabolites produced by probiotic microorganisms and which have a beneficial effect on the health of the host at a nutritional, metabolic and immune level, for example and without limitation, metabolites such as vitamin K, short-chain fatty acids or neurotransmitters such as GABA, serotonin or acetylcholine.

The term "parabiotic" as used in the present invention refers to non-viable material of microbial origin that has been shown to have human and/or animal health benefits. This non-viable material could include for example and without limitation cellular components such as teichoic acids, peptidoglycans or surface proteins.

The term "psychobiotic" as used in the present invention refers to probiotics, prebiotics and other dietary strategies that influence the microbiota (for example, some fibres present in fruits and vegetables) and which exert a mental health benefit.

Another aspect of the present invention relates to the use of the strain of the invention, the derived strain, or the components derived from it/them, or the composition of the invention, for manufacturing or producing a medicament, a nutritional composition or a food. The term "medicament" has been defined earlier and is applicable to the present aspect of the invention.

### Uses of the invention

As mentioned earlier, the inventors have demonstrated that B. *longum* strain CECT 30763 has the ability to prevent and/or attenuate mood disorders such as depressive or anxiogenic behaviour. Therefore, in another aspect, the present invention relates to the strain of the invention, a strain derived from it, a cellular component, metabolite, secreted molecule or any of the combinations thereof, obtained from the strain of the invention, the composition of the invention, including the pharmaceutical composition or the nutritional composition of the invention, for use in the prevention and/or treatment of at least one mood disorder in a subject, as well as their joint presence as comorbidities.

**In** the present invention, "subject" is understood to be any animal, preferably a mammal, more preferably a primate, in particular, a human, of any race, sex, or age. In a preferred embodiment, alone or in combination with the other preferred embodiments, the subject is a human. In some embodiments, alone or in combination with other embodiments, the subject is a subject exhibiting stress.

In the present invention, the term "treatment" refers to fighting the effects of the disease or pathological condition of interest in a subject (preferably mammal, and more preferably human) that includes:
(i) inhibiting the disease or pathological condition, that is, stopping its development;
(ii) alleviating the disease or pathological condition, that is, causing the remittance of the disease or pathological condition or the symptoms thereof;
(iii) stabilising the disease or pathological condition.

In the present invention, the term "prevention" refers to avoiding the onset of the disease, that is, preventing the disease or pathological condition from appearing in a subject (preferably mammal, and more preferably a human), in particular, when said subject has a predisposition for the pathological condition.

In the present invention, mood disorders can be defined as conditions that affect the mental/emotional state of a subject and may have an impact on their behaviour, and include, but are not limited to, depression, anxiety disorders, major depression, atypical depression, typical or melancholic depression, psychotic depression, catatonic depression, prenatal and postpartum depression, bipolar disorder, seasonal affective disorder, dysthymia, depressive personality disorder, double depression, unspecified depressive disorder, recurrent brief depressive disorder, minor depression, mood disorder induced by substance abuse or drug use.

Another preferred embodiment relates to the strain of the invention, a strain derived from it, a cellular component, metabolite, secreted molecule or any of the combinations thereof, obtained from the strain of the invention or the derived strain, the composition of the invention, including the pharmaceutical composition or the nutritional composition of the invention, for use in the prevention and/or treatment of at least two, three, four, five or a plurality of mood disorders in a subject. Mood disorders may also occur simultaneously or sequentially.

In a more preferred embodiment, the mood disorder is selected from the list consisting of depression, anxiety or anxiety disorder, major depression, atypical depression, typical or melancholic depression, psychotic depression, catatonic depression, prenatal and postpartum depression, bipolar disorder, seasonal affective disorder, stress, dysthymia, depressive personality disorder, double depression, unspecified depressive disorder, recurrent brief depressive disorder, minor depression, mood disorder induced by substance abuse, mood disorder induced by drug use, and any combination thereof. More preferably, the mood disorder is depression and/or anxiety. More preferably, when the mood disorder is stress, the stress is chronic stress, even more preferably chronic social stress, or acute stress.

Anxiety is an emotion characterised by feelings of tension, worrying thoughts and physical changes, such as increased blood pressure. Anxiety can be experienced not only by humans, but also by other animals, particularly other non-human mammals.

Anxiety disorders are the most common mental disorders in humans and affect nearly 30% of adults at some point in their lives. Anxiety disorders include, but are not limited to, generalised anxiety disorder, panic disorder, specific phobia, agoraphobia, social anxiety disorder (formerly called social phobia), noise aversion or separation anxiety disorder.

**In** the present invention, the term "anxiety disorders" includes not only the anxiety disorders described above, but also related conditions, including but not limited to post-traumatic stress disorder (PTSD); acute stress disorder; obsessive-compulsive disorder or adjustment disorders.

Moreover, depression is a highly prevalent mental illness that the healthcare system has to deal with. Like anxiety, depression can be experienced not only by humans, but also by other animals, particularly other non-human mammals.

**In** the present invention, the term "depression" refers to a clinical disorder that includes a predominantly sad or depressed mood and may be accompanied by other psychological and/or physical symptoms, presenting optionally.

Depression and/or anxiety affect social functioning and productivity, including work productivity, and increase the risk of suicide and other chronic diseases such as cardiovascular disease, stroke, diabetes, and obesity, as a depressive or anxiety-related disorder.

**In** particular, depression is thought to occur in up to 60% of people with anxiety disorders.

Depressive symptoms are associated with future impairments in mobility and functioning, and with higher medical costs.

Furthermore, the *B*. *longum* strain CECT 30763 is capable of improving the neuroendocrine response, as shown in Example 4, in which treatment with *B*. *longum* CECT 30763 in the animal model of stress-induced depression reduced plasma levels of corticosterone and dopamine. The *B*. *longum* strain CECT 30763 also reduces mediators of chronic stress-induced inflammation (CXCL9, CXCL10, CCL2, IL-6 and TNF-α) and increases the proportion of regulatory T cells in the intestine and spleen after acute and chronic stress (Example 5, Figures 6A, 6B and 6C). Therefore, in another aspect, the present invention relates to the strain of the invention, a strain derived from it, a cellular component, metabolite, secreted molecule or any of the combinations thereof, obtained from the strain of the invention or the derived strain, the composition of the invention, including the pharmaceutical composition or the nutritional composition of the invention, for use in improving the neuroendocrine response in a subject, preferably for use in improving the neuroendocrine response comorbid with at least one mood disorder. Examples used to define mood disorders have been mentioned earlier, and both these and their preferred embodiments are also applicable to the present aspect of the invention.

Furthermore, the strain of the invention and products derived therefrom also have applicability in relation to the prevention and/or treatment of cardiometabolic disorders.

Therefore, another aspect of the invention relates to the strain of the invention, a strain derived from it, a cellular component, metabolite, secreted molecule or any of the combinations thereof, obtained from the strain of the invention or the derived strain, the composition of the invention, including the pharmaceutical composition or the nutritional composition of the invention, for use in the prevention and/or treatment of at least one cardiometabolic disorder in a subject.

As in the other previous aspect, some preferred embodiments provide for the prevention and/or treatment of at least two, three, four, five or a plurality of cardiometabolic disorders in a subject. These disorders may present simultaneously or sequentially.

Cardiometabolic disorders are disorders often related to different interrelated factors such as hypertension, inflammation, elevated blood glucose levels, dyslipidemia, abdominal obesity and elevated triglycerides in one subject. These cardiometabolic disorders include cardiovascular or metabolic diseases or disorders.

Examples of cardiometabolic disorders include, but are not limited to, hyperlipidaemia, hyperglycaemia, metabolic syndrome, type 2 diabetes, obesity, arterial hypertension, coronary heart disease, or other cardiovascular disease. In a more preferred embodiment, alone or in combination with the other preferred embodiments, the cardiometabolic disorder is selected from the list consisting of: a cardiovascular disease, metabolic syndrome, type 2 diabetes, obesity, hyperlipidaemia, hyperglycaemia, and any combination thereof.

The strain of the invention is shown to exert protective effects on cardiometabolic health, by reducing oxidative stress in the heart (Example 6, Figure 11). Moreover, as mentioned, the strain of the invention is capable of reducing levels of CXCL9, CXCL10, CCL2, IL-6 and TNF-α, which are inflammatory mediators that are also increased in cardiometabolic pathologies. Furthermore, *B*. *longum* strain CECT 30763 also increased Tregs in the spleen and intestine after acute and chronic stress, indicating an anti-inflammatory effect (Example 5, Figures 6A, 6B and 6C).

Therefore, in another more preferred embodiment, alone or in combination with the other preferred embodiments, the cardiometabolic disorder comprises inflammation. More preferably, inflammation is mediated by at least one cytokine selected from the list consisting of CXCL9, CXCL10, CCL2, IL-6, TNF-α and any combination thereof, and/or by reduced Treg levels compared to reference values. "Reference values" is understood as Treg levels in healthy subjects.

The present invention also has applicability in relation to the prevention and/or treatment of liver disorders. Therefore, another aspect of the invention relates to the strain of the invention, a strain derived from it, a cellular component, metabolite, secreted molecule or any of the combinations thereof, obtained from the strain of the invention or the derived strain, the composition of the invention, including the pharmaceutical composition or the nutritional composition of the invention, for use in the prevention and/or treatment of at least one liver disorder or condition in a subject. As in previous aspects of the invention, some preferred embodiments provide for the prevention and/or treatment of at least two, three, four, five or a plurality of liver disorders in a subject. These disorders may present simultaneously or sequentially.

In the present invention, liver disorders are understood to be disorders or conditions that impair the normal function of the liver. Examples of liver disorder include, but are not limited to, fatty liver or steatosis , liver fibrosis, hepatocellular carcinoma, hepatic failure or hepatitis.

In another more preferred embodiment, alone or in combination with the other preferred embodiments, the liver disorder is selected from the list consisting of:hepatic steatosis , liver fibrosis, hepatitis, hepatocellular carcinoma, hepatic failure and any combination thereof.

In another aspect, the invention relates to the strain of the invention, a strain derived from it, a cellular component, metabolite, secreted molecule or any of the combinations thereof, obtained from the strain of the invention or the derived strain, or the composition of the invention, for use in the prevention and/or treatment of mood disorders and/or cardiometabolic disorders and/or liver disorders and/or in improving the neuroendocrine response in a subject.

Mood disorders, such as depression and/or anxiety, are associated with other psychiatric and non-psychiatric medical conditions (for example, cardiometabolic disorders, such as cardiovascular diseases, metabolic syndrome, diabetes and liver disorders, such as fatty liver orsteatosis liver fibrosis) and often present together.

In the present invention, the term "comorbidity" refers to the presence of one or more disorders in addition to the primary disease or disorder in the same subject. They may occur at the same time or one after the other. "Comorbidity" also implies that there may be an interaction between the diseases, worsening the course of each one and making treatment more difficult.

Therefore, in another aspect, the present invention relates to the strain of the invention, a strain derived from it, a cellular component, metabolite, secreted molecule or any of the combinations thereof, obtained from the strain of the invention or the derived strain, the composition of the invention, including the pharmaceutical composition or the nutritional composition of the invention, for use in the prevention and/or treatment of comorbid mood disorders in a subject.

Examples used to define mood disorders have been discussed above, and both these and their preferred embodiments are also applicable to the present aspect of the invention.

The mood disorders are preferably depression and anxiety.

In another aspect, the present invention relates to the strain of the invention, a strain derived from it, a cellular component, metabolite, secreted molecule or any of the combinations thereof, obtained from the strain of the invention or the derived strain, the composition of the invention, including the pharmaceutical composition or the nutritional composition of the invention, for use in the prevention and/or treatment of cardiometabolic comorbidities in a subject. The expression "cardiometabolic comorbidities" refers to one or more cardiometabolic disorders being present in a subject in addition to the primary disorder in the present invention, wherein said primary disorder is at least one mood disorder. Therefore, the present aspect relates to use in the prevention and/or treatment of one or more cardiometabolic disorders comorbid with at least one mood disorder in a subject.

Examples used to define cardiometabolic disorders have been mentioned earlier, and both these and their preferred embodiments are also applicable to the present aspect of the invention.

In another particular embodiment, the cardiometabolic disorder comprises inflammation.

In another aspect, the present invention relates to the strain of the invention, a strain derived from it, a cellular component, metabolite, secreted molecule or any of the combinations thereof, obtained from the strain of the invention or the derived strain, the composition of the invention, including the pharmaceutical composition or the nutritional composition of the invention, for use in the prevention and/or treatment of liver comorbidities in a subject. The expression "liver comorbidities" refers to one or more liver disorders being present in a subject in addition to the primary disorder in the present invention, wherein said primary disorder is at least one mood disorder. Therefore, the present aspect relates to use in the prevention and/or treatment of one or more liver disorders comorbid with at least one mood disorder in a subject.

Examples used to define liver disorders have been mentioned earlier, and both these and their preferred embodiments are also applicable to the present aspect of the invention.

**In** another aspect, the present invention relates to the strain of the invention, a strain derived from it, a cellular component, metabolite, secreted molecule or any of the combinations thereof, obtained from the strain of the invention or the derived strain, the composition of the invention, including the pharmaceutical composition or the nutritional composition of the invention, for use in the prevention and/or treatment of cardiometabolic and liver comorbidities in a subject. As described earlier, when "cardiometabolic comorbidities" and "liver comorbidities" were defined, they refer to one or more cardiometabolic disorders and one or more liver disorders, respectively, being present in a subject in addition to the primary disorder in the present invention, wherein said primary disorder is at least one mood disorder. That is, the present aspect of the invention relates to use in the prevention and/or treatment of one or more liver disorders and one or more cardiometabolic disorders comorbid with at least one mood disorder in a subject.

Furthermore, the strain of the invention, strain derived from it, cellular component, metabolite, secreted molecule or any of the combinations thereof, obtained from the strain of the invention or the derived strain, or the composition of the invention, can be used as an adjuvant

**In** the present invention, "adjuvant " is understood to be a compound that helps to improve the effectiveness or efficiency of other medicaments for the treatment and/or prevention of a disorder or disease, which would allow its dose and/or frequency of administration to be reduced or its efficacy to be enhanced by administering a formulation of the strain of the invention with complementary mechanisms of action.

Therefore, in another aspect, the invention relates to the strain of the invention, a strain derived from it, a cellular component, metabolite, secreted molecule or any of the combinations thereof, obtained from the strain of the invention or the derived strain, or the composition of the invention, for use as an adjuvant in the treatment and/or prevention of mood disorders and/or cardiometabolic disorders and/or liver disorders and/or in improving the neuroendocrine response in a subject.

Examples used to define mood disorders, cardiometabolic disorders or liver disorders have been mentioned earlier, and both these and their preferred embodiments are also applicable to the present aspect of the invention.

**In** another aspect, the invention relates to the strain of the invention, a strain derived from it, a cellular component, metabolite, secreted molecule or any of the combinations thereof, obtained from the strain of the invention or the derived strain, or the composition of the invention, for use as an adjuvant in the treatment and/or prevention of comorbid mood disorders.

**In** another aspect, the invention relates to the strain of the invention, a strain derived from it, a cellular component, metabolite, secreted molecule or any of the combinations thereof, obtained from the strain of the invention or the derived strain, or the composition of the invention, for use as an adjuvant in the treatment and/or prevention of cardiometabolic comorbidities and/or liver comorbidities and/or improvement of comorbid neuroendocrine response in a subject.

**In** another aspect, the invention relates to the strain of the invention, a strain derived from it, a cellular component, metabolite, secreted molecule or any of the combinations thereof, obtained from the strain of the invention or the derived strain, or the composition of the invention, for use as an adjuvant in the treatment and/or prevention of one or more cardiometabolic disorders and one or more liver disorders comorbid with at least one mood disorder in a subject.

### Prevention and/or treatment methods

**In** another aspect, the invention relates to a method for the prevention and/or treatment of at least one mood disorder in a subject, comprising the administration of the strain of the invention, a strain derived from it, a cellular component, metabolite, secreted molecule or any of the combinations thereof, obtained from the strain of the invention or the derived strain, or the composition of the invention.

**In** another aspect, the invention relates to a method for the prevention and/or treatment of at least one cardiometabolic disorder in a subject, comprising the administration of the strain of the invention, a strain derived from it, a cellular component, metabolite, secreted molecule or any of the combinations thereof, obtained from the strain of the invention or the derived strain, or the composition of the invention.

**In** another aspect, the invention relates to a method for the prevention and/or treatment of at least one liver disorder or condition in a subject, comprising the administration of the strain of the invention, a strain derived from it, a cellular component, metabolite, secreted molecule or any of the combinations thereof, obtained from the strain of the invention or the derived strain, or the composition of the invention.

**In** another aspect, the invention relates to a method for the prevention and/or treatment of comorbid mood disorders in a subject, comprising the administration of the strain of the invention, a strain derived from it, a cellular component, metabolite, secreted molecule or any of the combinations thereof, obtained from the strain of the invention or the derived strain, or the composition of the invention.

**In** another aspect, the invention relates to a method for the prevention and/or treatment of cardiometabolic comorbidities in a subject, comprising the administration of the strain of the invention, a strain derived from it, a cellular component, metabolite, secreted molecule or any of the combinations thereof, obtained from the strain of the invention or the derived strain, or the composition of the invention. The expression "cardiometabolic comorbidities" refers to one or more cardiometabolic disorders being present in a subject in addition to the primary disorder in the present invention, wherein said primary disorder is at least one mood disorder. Therefore, the present aspect relates to the method in the prevention and/or treatment of one or more cardiometabolic disorders with at least one mood disorder in a subject.

**In** another aspect, the invention relates to a method for the prevention and/or treatment of liver comorbidities in a subject, comprising the administration of the strain of the invention, a strain derived from it, a cellular component, metabolite, secreted molecule or any of the combinations thereof, obtained from the strain of the invention or the derived strain, or the composition of the invention. The expression "liver comorbidities refers to one or more liver disorders being present in a subject in addition to the primary disorder in the present invention, wherein said primary disorder is at least one mood disorder. Therefore, the present aspect relates to the method in the prevention and/or treatment of one or more liver disorders with at least one mood disorder in a subject.

**In** another aspect, the invention relates to a method for the prevention and/or treatment of cardiometabolic and liver comorbidities in a subject, comprising the administration of the strain of the invention, a strain derived from it, a cellular component, metabolite, secreted molecule or any of the combinations thereof, obtained from the strain of the invention or the derived strain, or the composition of the invention. The present aspect relates to the method in the prevention and/or treatment of one or more liver disorders and one or more cardiometabolic disorders with at least one mood disorder in a subject.

The terms used to define the prevention and/or treatment methods of the invention have been defined and explained in previous aspects of the invention, with both these and their preferred embodiments being applicable to them.

### DESCRIPTION OF THE FIGURES

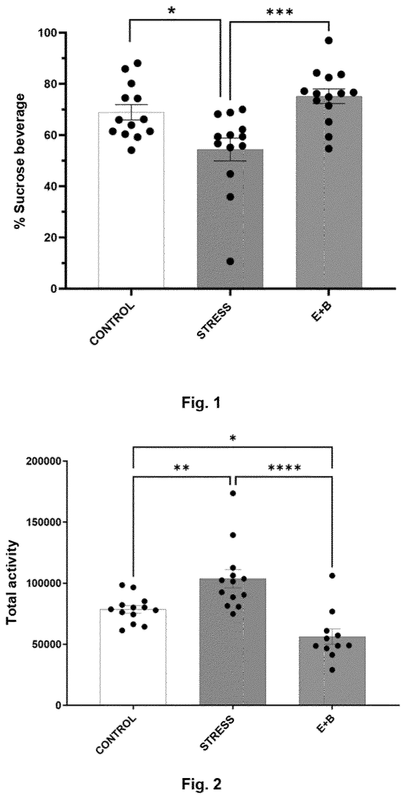

**Figure 1****. Evaluation of the effect of the administration of *B. longum* CECT 30763 (1x10⁹ cfu/day) in C57BL/6 mice (n=13 (C), 13 (E), 14 (E+B)) exposed to chronic social stress in the sucrose preference test.** Data are expressed as the percentage of sucrose intake relative to total volume intake and standard error. Statistically significant differences were established by applying a Kruskal-Wallis analysis followed by Dunn's multiple comparison test (*=p<0.05, ***=p<0.001). C, control mice; E, mice subjected to chronic stress without treatment; E+B, mice subjected to chronic stress mice treated with *B*. *longum* CECT 30763.
**Figure 2****. Evaluation of the effect of the administration of *B. longum* strain CECT 30763 (1x10⁹ cfu/day) in C57BL/6 mice (n=13 (C), 13 (E), 11 (E+B)) exposed to chronic social stress in the tail suspension test.** Data are expressed in seconds and standard error. Statistically significant differences were established by applying one-way ANOVA followed by the post hoc Tukey test (*=p<0.05, **=p<0.01, ****=p<0.0001). C, control mice; E, mice subjected to chronic stress without treatment ; E+B, mice subjected to chronic stress treated with *B. longum* CECT 30763.

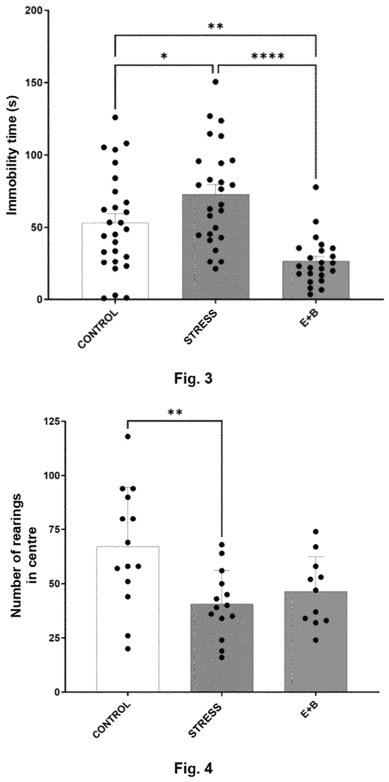

**Figure 3****. Evaluation of the effect of the administration of *B. longum* CECT 30763 (1x10⁹ cfu/day) in C57BL/6 mice (n=27 (C), 26 (E), 23 (E+B)) exposed to chronic social stress in the forced swim test.** Data are expressed in seconds and standard error. Statistically significant differences were established by applying one-way ANOVA followed by the post hoc Tukey test (*=p<0.05, **=p<0.01, ****=p<0.0001). C, control mice; E, mice subjected to chronic stress without treatment; E+B, mice subjected to chronic stress treated with *B. longum* CECT 30763.
**Figure 4****. Evaluation of the effect of the administration of *B. longum* CECT 30763 (1x10⁹ cfu/day) in C57BL/6 mice (n=14 (C), 14 (E), 11 (E+B)) exposed to chronic social stress in open field tests.** Data are expressed as number of rearing or upright exploratory movements and standard error. Statistically significant differences were established by applying a Kruskal-Wallis analysis followed by Dunn's multiple comparison test (**=p<0.01). C, control mice; E, mice subjected to chronic stress without treatment ; E+B, mice subjected to chronic stress treated with *B*. *longum* CECT 30763.

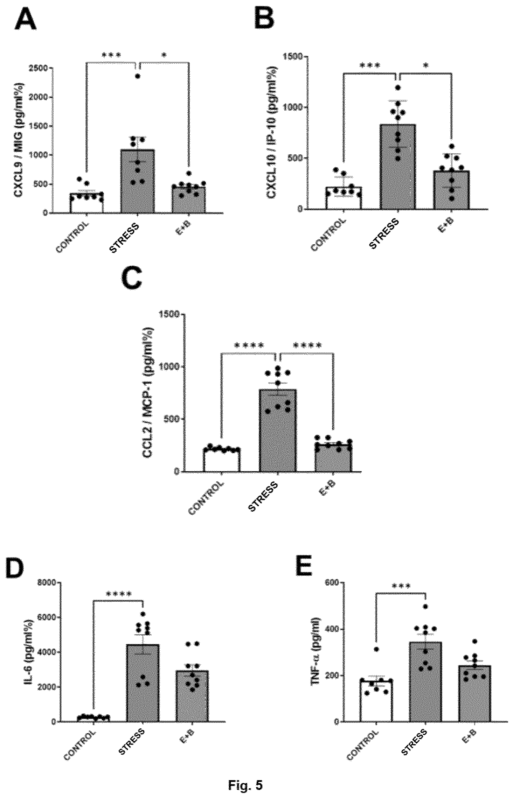

**Figure 5****. Evaluation of the effect of the administration of *B. longum* CECT 30763 (1x10⁹ cfu/day) in C57BL/6 mice (n=8-9/group) exposed to chronic social stress on the plasma immune system.** Plasma levels of chemokines and cytokines CXCL9 (A), CXCL10 (B), CCL2 (C), IL-6 (D), TNF-α (E). Data are expressed in picograms (pg) per millilitre (ml) and standard error. Statistically significant differences were established by applying a Kruskal-Wallis analysis followed by Dunn's multiple comparison test (*=p<0.05, ***=p<0.001, ****=p<0.0001). C, control mice; E, mice subjected to chronic stress without treatment ; E+B, mice subjected to chronic stress treated with *B. longum* CECT 30763.
**Figure 6****. Evaluation of the effect of the administration of *B. longum* CECT 30763 (1x10⁹ cfu/day) in C57BL/6 mice (n=8-10/group) exposed to acute and chronic social stress on the immune system in the spleen and small intestine.** Levels of Treg lymphocytes in the spleen (A) and small intestine (B) in mice exposed to chronic stress and levels of Treg lymphocytes in the spleen (C) in mice exposed to acute stress. Data are expressed in picograms (pg) per millilitre (ml) and standard error. Statistically significant differences were established by applying one-way ANOVA followed by the post hoc Tukey test (*=p<0.05, **=p<0.01, ****=p<0.0001). C, control mice; E, mice subjected to chronic stress without treatment; E+B, mice subjected to chronic stress treated with *B*. *longum* CECT 30763.

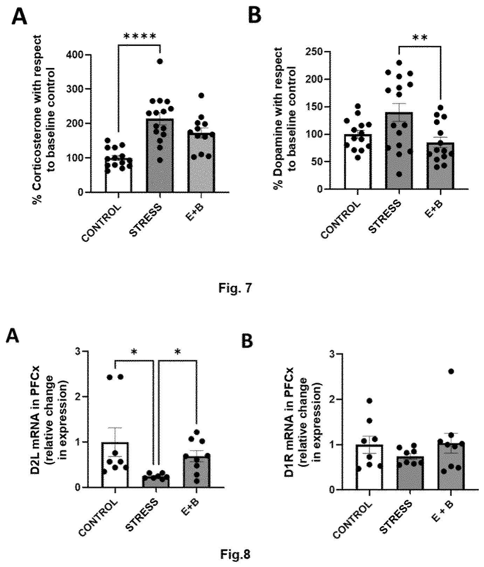

**Figure 7****. Evaluation of the effect of the administration of *B. longum* CECT 30763 (1x10⁹ cfu/day) in C57BL/6 mice (n=12-16/group) exposed to chronic social stress on plasma levels of cortisone and dopamine.** Plasma levels of corticosterone (A) and dopamine (B). Data are expressed as a percentage relative to the control group and standard error. Statistically significant differences were established by applying one-way ANOVA followed by the post hoc Tukey test (**=p<0.01, ****=p<0.0001). C, control mice; E, mice subjected to chronic stress without treatment; E+B, mice subjected to chronic stress treated with B. *longum* CECT 30763.
**Figure 8****. Evaluation of the effect of the administration of *B. longum* CECT 30763 (1x10⁹ cfu/day) in C57BL/6 mice (n=7-9/group) exposed to chronic social stress on the expression of dopamine receptors in the prefrontal cortex of the brain.** Relative mRNA expression of dopamine D2L (A) and D1R (B) receptors in the prefrontal cortex (PFCx). The data are expressed on an individual basis, where each point and the histograms represent the mean and the standard error. Statistically significant differences were established by applying a Kruskal-Wallis analysis followed by Dunn's multiple comparison test (*=p<0.05). C, control mice; E, mice subjected to chronic stress without treatment; E+B, mice subjected to chronic stress treated with B. *longum* CECT 30763.
**Figure 9****. Evaluation of the effect of the administration of *B. longum* CECT 30763 (1x10⁹ cfu/day) in C57BL/6 mice (n=6-9/group) exposed to chronic social stress on the expression of profibrotic genes in the liver.** Relative mRNA expression of liver fibrosis-related genes TGFβ1 (A), Col1a1 (B), Col3a1 (C). The data are represented on an individual basis, where each point and the histograms represent the mean and the standard error. Statistically significant differences were established by applying a one-way ANOVA test with a Bonferroni post-test (*p<0.05; ** p>0.01). C, control mice; E, mice subjected to chronic stress without treatment ; E+B, mice subjected to chronic stress treated with B. *longum* CECT 30763.
**Figure 10****. Evaluation of the effect of the administration of *B. longum* CECT 30763 (1x10⁹cfu/day) in C57BL/6 mice (n=8-9/group) exposed to chronic social stress on the expression of genes involved in fatty acid metabolism in the liver.** Relative mRNA expression of ACC1 (A), DGAT (B), CPT1a (C) genes, and the DGAT/CPT1 ratio (D). The data are represented on an individual basis, where each point and the histograms represent the mean and the standard error. Statistically significant differences were established by applying a one-way ANOVA test with a Bonferroni post-test or Student's t-test between two groups when indicated (*p<0.05). C, control mice; E, mice subjected to chronic stress without treatment; E+B, mice subjected to chronic stress treated with B. *longum* CECT 30763.
**Figure 11****. Evaluation of the effect of the administration of *B. longum* CECT 30763 (1x10⁹ cfu/day) on oxidative stress damage in C57BL/6 mice (n=9 (C), 9 (E), 7 (E+B)) exposed to chronic social stress.** Percentage of 8-hydroxy-2'-deoxyguanosine (8-OHdG) antibody-positive tissue area versus total tissue area. The data are represented on an individual basis, where each point and the histograms represent the mean and the standard error of the mean. Statistically significant differences were established by applying a one-way ANOVA test with a Bonferroni post-test (*p<0.05; **p<0,01). C, control mice; E, mice subjected to chronic stress without treatment ; E+B, mice subjected to chronic stress treated with *B. longum* CECT 30763.

### EXAMPLES

Next, the invention will be illustrated by means of assays carried out by the inventors which demonstrate the effectiveness of the invention.

### EXAMPLE 1. Isolation and identification of B. longum strain CECT 30763

*B. longum* strain CECT 30763 was isolated and identified from the faeces of healthy volunteers on MRS agar + 0.05% L-cysteine (MRS-cis). Serial dilutions of stool samples were made in PBS-1x + 0.05% L-cysteine and cultured on MRS-cis plates in anaerobic conditions at 37°C for 48 hours. Isolated colonies were identified by Gram staining and subsequent colony PCR sequencing.

Two different genes were used to identify the species and different strains of B. *longum.* The 16S rRNA gene (1.5 Kb) was amplified with primers 27F (SEQ ID NO 1: 5'-AGAGAGTTTGATCCTGGCTCAG-3') and 1401R (SEQ ID NO 2: 5'-CGGTGTGTACAAGACCC-3') which allowed the genus and species to be identified, and the threonine-tRNA ligase (ThrS) gene was amplified with primers BifiThrS-F (SEQ ID NO 3: 5'-AAGGACGGCTTCTACTACTACGA-3') and BifiThrS-R (SEQ ID NO 4: 5'-AAGATCAGGTTGTGCATCGG-3') and, when used together with the above, was useful for strain differentiation. Both genes were sequenced by Sanger technology on an Applied Biosystems ABI 3730XL sequencer using both forward and reverse primers. Two nucleotide sequences were obtained for each gene (16S rRNA and ThrS), one sequenced with the direct primer and the other one sequenced with the reverse primer; using the PRABI-Doua web tool, a single amplicon is generated for each of the genes (SEQ ID NO: 5 and SEQ ID NO: 6, respectively). By comparing the sequences obtained from the 16S rRNA with those of the NCBI database and the BLASTn algorithm, the identification of the species was obtained with a percentage identity of 100%. Next, both genes were aligned separately for subsequent concatenation with MAFFT software, obtaining a single nucleotide sequence consisting of the 16S rRNA gene, 20 ambiguous nucleotides (N) and the ThrS gene of each of the strains used to carry out the analysis. The phylogeny was generated using MEGAX software. After entering the sequences into the programme, an alignment was performed by means of ClustalW+Muscle; using the maximum likelihood statistical model, the T92 (Tamura-3-parameter) nucleotide substitution model was selected. Lastly, the analysis was carried out using the Bootstrap method, with 1000 replicas. By creating a dendrogram, it was confirmed that the strain object of the patent *(B. longum* G121 or B. *longum* CECT 30763) differs from other strains of the same species.
>B.longum_G121_16S-rRNA (SEQ ID NO: 5): 
GCAGTCGAACGGGATCCACCGGGCTTTGCTTGGTGGTGAGAGTGGCGAACGGGT
GAGTAATGCGTGACCGACCTGCCCCATACACCGGAATAGCTCCTGGAAACGGGT
GGTAATGCCGGATGCTCCAGTTGATCGCATGGTCTTCTGGGAAAGCTTTCGCGGT
ATGGGATGGGGTCGCGTCCTATCAGCTTGACGGCGGGGTAACGGCCCACCGTG
GCTTCGACGGGTAGCCGGCCTGAGAGGGCGACCGGCCACATTGGGACTGAGAT
ACGGCCCAGACTCCTACGGGAGGCAGCAGTGGGGAATATTGCACAATGGGCGCA
AGCCTGATGCAGCGACGCCGCGTGAGGGATGGAGGCCTTCGGGTTGTAAACCTC
TTTTATCGGGGAGCAAGCGAGAGTGAGTTTACCCGTTGAATAAGCACCGGCTAAC
TACGTGCCAGCAGCCGCGGTAATACGTAGGGTGCAAGCGTTATCCGGAATTATTG
GGCGTAAAGGGCTCGTAGGCGGTTCGTCCCGTCCGGTGTGAAAGTCCATCGCTT
AACGGTGGATCCGCGCCGGGTACGGGCGGGCTTGAGTGCGGTAGGGGAGACTG
GAATTCCCGGTGTAACGGTGGAATGTGTAGATATCGGGAAGAACACCAATGGCGA
AGGCAGGTCTCTGGGCCGTTACTGACGCTAAGGAGCGAAAGCGTGGGGAGCGAA
CAGGATTAGATACCCTGGTAGTCCACGCCGTAAACGGTGGATGCTGGATGTGGG
GCCCGTTCCACGGGTTCCGTGTCGGAGCTAACGCGTTAAGCATCCCGCCTGGGG
AGTACGGCCGCAAGGCTAAAACTCAAAGAAATTGACGGGGGCCCGCACAAGCGG
CGGAGCATGCGGATTAATTCGATGCAACGCGAAGAACCTTACCTGGGCTTGACAT
GTTCCCGACGGTCGTAGAGATACGGCTTCCCTTCGGGGCGGGTTCACAGGTGGT
GCATGGTCGTCCTCAGCTCGTGTCGTGAAATGTTGGGTTAAGTCCCGCAACGAGC
GCAACCCCCGCCCCGTGTTGCCAGCGGATTATGCCGGGAACTCACGGGGAACCG
CCCGGGTTAACTCGGAGGAAGGTGGGGATGACGTCAAATCATCATGCCCCTTAC
GTCCAGGGCTTCACGCATGCTACAATGGCCGGTACAACGGGATGCGACGCGGCG
ACGCGGAGCGGATCCCTGAAAACCGGTCTCAGTTCGGATCGCAGTCTGCAACTC
GACTGCGTGAAGGCGGAGTCGCTAGTAATCTGCGAAT
>B.longum_G121_ ThrS (SEQ ID NO: 6):

CATCGGGCAGTTCATCGGCTTGAGGTAGTAGTCGGCGCCCGGCTTGGTGATGTT
GCCGTCCGCGTCCTTCTCCTCGTCGAGGTGCATGGGCGGGTACATGCCGTCCTT
GTACCAGTGCAGGTGGCCGGAGGTCTCGTACAGGCCGCCCTTGGTGATGTGCGG

GGTCTGCACGAAGCTGTAATGGTGCTTGCGGTGCATCTCACGGGAGTAGATCCTC
CATAGCGTTGATCACGGCGGCACCCTTCGGGTGGAACACGGCCAGGCCGGGGC
CAATTTCATCCGGGAAGGAGAACAGGTCCATCTCGGCGCCGAGCTTGCGGTGAT
CGCGCTTGGCGGCTTCTTCCAGGCGGGTCTGGTAGGCCTTGAGGTCTTCCTTGG
TGGCCCATGCGGTGCCGTAGACGCGCTGCATGGTCGGGTTCTTCTCGGAACCAC
GCCAGTAGGCGGCGGCGGAACGCTCGATTTTGAAGGCCTTGATGTAGCGGGTGT
TCGGCAGGTGCGGGCCGCGGCACAGGTCCTTCCAAACCACATTGCCATCACGGT
CTACGTTGTCGTAGAAGCTGAGCTCCTTTTCGGAGATCTCGGTGGCGGCGGCCG
GGTCGAGGTGTGCTTCCTTGTCTTCGATCAGTTCGATCTTAAAGGCTGGTCGGC
CTCTTCCTTCAGAGCCTCTTCCTCGGTCACGGAACGGCGGCGGAAGGACTGGGA
GGACTTGATGATGCGCTGCATGCGCTTCTCGATGTCCTTGAGGTCGTTCGGGGTG
AAGGGCTGGTCGACCTGTAAAGTCGTAGTA

### EXAMPLE 2. Effects of B. longum strain CECT 30763 on behaviour in a model of social stress-induced depression.

### Production of B. lonqum strain CECT 30763

The B. *longum* strain CECT 30763 was grown in MRS liquid medium at 37°C in anaerobic conditions, using an anaerobic chamber with a gas mixture of 10% CO₂, 10% H₂, 80% N₂ (Bactron 300-2, Shellab, Cornelius, OR, USA). Cells were collected by centrifugation (12,000 g for 10 min at 4°C) and washed with phosphate buffered saline (PBS, 130 mM sodium chloride), 10 mM sodium phosphate, pH 7.4) supplemented with 0.05% L-cysteine. Bacteria were resuspended in PBS + 0.05% L-cysteine + 10% glycerol and aliquots of these suspensions were immediately frozen in liquid nitrogen and stored at - 80°C until use. Cell viability of the cultures was determined by culture on MRS agar plates; the viability of the cultures after storage at -80°C ranged from 98-99% live cells per ml of culture. Aliquots were thawed daily for administration to prevent deviations in viability of the culture during the study.

### Development of the animal model of acute and chronic social stress and sample collection

Male C57BL/ 6 mice that arrived at adolescent age were used (postnatal day 32, Charles River, Les Oncins, France). They were kept under controlled temperature (23°C), relative humidity (40-50%) and 12-hour light/dark cycle conditions and fed a standard diet (D12450K, Research diet, Brogaarden, Denmark) for 7 weeks (the first week they were quarantined in a room set up to prevent possible zoonotic diseases). Mice were randomly divided into 3 experimental groups (n=10). Control group (C), stress group (E), group treated with *B*. *longum* CECT 30763 (E+B). Mice in the *B*. *longum* group were treated with a daily dose of the bacterium at 1x10⁹ colony-forming units [cfu] suspended in PBS + 0.05% L-cysteine + 10% glycerol. The carrier or placebo (PBS + 0.05% L-cysteine + 10% glycerol) was administered equally to both the control and stress groups. Treatment or placebo was administered for 6 weeks. After these 6 weeks, a subgroup was used for behavioural tests and another subgroup was sacrificed by cervical dislocation to obtain samples, including blood, intestine, brain, faecal content and faeces. Other experimental groups were sacrificed after the first exposure to acute stress in order to analyse effects at earlier stages of the traumatic situation.

### Model of acute and chronic social stress

An animal model of social defeat based on the resident-intruder paradigm was used to induce chronic social stress. **In** this model, one of the two animals (the resident) was allowed to establish territoriality in its own crate. Next, the mice under study (intruders), in our case male C57BL/6 mice, were introduced one by one into the cage of the resident mouse. For this purpose, more aggressive adult males older than the mice under study (5 weeks older) were used as resident mice. These mice were from the CD-1 strain (Charles River, Les Oncins, France) and were previously isolated and trained to be more aggressive. For 10 consecutive days (for the model of chronic stress) or for 1 day (for the model of acute stress), agonistic encounters were performed (introduction of a naïve mouse into the resident's cage for 5 minutes) in which physical contact between them was allowed and in which the intruder mouse suffered a high degree of stress (reflected in the production of high levels of corticosterone). The agonistic encounters took place in a neutral room and not in the animal facility where they were typically housed. The experimental mice (intruders) showed evasive or flight behaviour, as well as defence/submission behaviour after suffering aggression (threat/attack) from their opponent. The criterion used to define that an animal had been defeated was the adoption of a specific posture signifying defeat. It is characterised by an upright submissive posture with limp forelegs, its head tilted upwards and its ears retracted [ Can, A., et al., The tail suspension test. J Vis Exp, 2012(59): p. e3769.].

The control group was not exposed to social defeat; however, all mice in this group were placed for 5 minutes in a cage exactly like the ones used for the agonistic encounters. For 5 minutes they explored the cage without contact with any opponent.

### 3% Sucrose Preference Test (SPT)

The 3% sucrose preference test was performed to assess hedonic/anhedonic behaviour associated with depressive behaviour. Anhedonic behaviour (inability to feel pleasure) is considered one of the clearest symptoms of depression. Various animal studies have shown that depressed animals consume less water containing 3% sucrose, which is considered to be anhedonic behaviour.

The test consists of depriving the animals of water for 12 hours and then exposing them to two options, either water or water with 3% sucrose dissolved in it.

The sucrose and water bottles were switched during the 2-hour test period to ensure that there was no effect related to site preference. The 3% sucrose intake amount during these 2 hours would indicate hedonic/anhedonic behaviour. Lower sucrose intake would indicate anhedonia. Sucrose preference was calculated as the percentage of sucrose intake in relation to the total amount of liquid consumed and corrected for body weight.

The results (Figure 1) indicate that stressed animals take in significantly less 3% sucrose than control mice (p<0.05), indicating anhedonic and, therefore, depressive behaviour. Treatment with B. *longum* CECT 30763 significantly reduces anhedonia (p<0.001), indicating its ability to reduce depressive behaviour in this test.

### Tail suspension test

Mice were suspended on the edge of a table with adhesive tape placed about 1 cm from the tip of the tail in a position where they could not escape or cling to nearby surfaces. Behaviour aimed at trying to escape, as well as the immobility time for 5 minutes, were quantified. The duration of activity and immobility was recorded during the 5 minutes that the test lasted. This test is widely used to assess depressive behaviour in mice [Can, A., et al., The tail suspension test. J Vis Exp, 2012(59): p. e3769].

The results (Figure 2) indicate that stressed animals show a significant increase in overall activity compared to control animals (p<0.01). Treatment with B. *longum* CECT 30763 (E+B) significantly normalises this altered behaviour in the stressed or E group (p<0.001), indicating its effectiveness in restoring depressive behaviour.

### Forced swimming test

To perform this test (Figure 3), a single mouse was placed in the water-filled beaker for a period of 5 minutes and its behaviour was recorded with software (Smart 2.0, Panlab). The time that the mice are either moving or immobile was analysed.

Results showed that stressed mice move significantly more (p<0.05) than control mice and treatment with B. *longum* CECT 30763 normalises this behaviour (p<0.0001), suggesting its effectiveness in restoring depressive behaviour disorders.

### Open field

This test measures activity and anxiety in an open field. It consists of placing the animals in an open box for 10 minutes. It is considered that the less activity they show in the centre of the open field, the more anxiety the animals exhibit.

The results of this test (Figure 4) indicate that stressed animals performed less rearing (the rodent stands on its two hind legs) in the centre than control mice (p<0.01). Treatment with *B*. *longum* CECT 30763 tends to normalise this value by removing significance.

Overall, the results of these behavioural tests show that treatment with *B*. *longum* CECT 30763 restores depressive and anxiogenic behaviour in mice that have been exposed to chronic social stress, which are models of these pathologies.

### EXAMPLE 3. Effects of B. longum strain CECT 30763 on the immune response in an animal model of social stress-induced depression.

The immune system response was analysed in plasma, spleen and small intestine by flow cytometry in the animal model of acute and chronic social stress-induced depression. Flow cytometry was used to assess this response. Flow cytometry is an analytical method that allows the rapid measurement of certain physical and chemical characteristics of cells or particles suspended in liquid that individually produce a signal when interfered with by a light source.

To measure cytokines and chemokines in plasma, plasma was obtained from blood with EDTA and centrifuged at 3500 rpm for 10 minutes at 4°C. A multiplex kit (LEGENDplex^{™} Mouse Inflammation Panel) specific for fluorescently labelling cytokines and chemokines was used so that they could be quantified simultaneously by flow cytometry, following the manufacturer's instructions. The labelled samples were analysed by flow cytometry with a FORTESSA kit (Becton Dickinson, BD LSR-FORTESSA SORP).

Cells from the small intestine were isolated according to the procedure previously described by the present authors (López-Almela et al., Bacteroides uniformis combined with fiber amplifies metabolic and immune benefits in obese mice. Gut Microbes.2021, 13(1):p. 1). The cells isolated and suspended in FACS buffer (PBS with 0.5% BSA) were incubated with different immune markers for 30 minutes at 4°C in the dark to measure lymphocyte subpopulations (specifically regulatory T-lymphocytes, also known as Tregs).

For the spleen, single-cell suspensions were prepared by pressing the tissues through a 100 µm cell strainer (Sarstedt placed in a 50 ml tube using the plunger of a 1 ml syringe and washing the strainer with sterile 1X PBS supplemented with 5% FBS (FACS 5%)). The samples were centrifuged for 5 minutes at 300 g. The red blood cells were lysed by incubation for 10 minutes at 37°C with 5 ml of 1X RBC lysis buffer (eBioscience). The immune cells were washed with 5% FACS.

The spleen and intestine samples were stained on FACS 5% with a previous incubation with CD16/CD32 anti-mouse (clone 2.4 G2, Bioscience). Treg cells were determined with: CD45, AF700, CD19 PE-Cy7, CD4 PE, CD3 PerCp Cy5.5, CD 8 FITC, CD25 APC, Foxp3 BV421 (BD). For intracellular labelling, the Mouse Foxp3 Buffer Set kit (BD) was used. The samples were purchased from BD LSR-FORTESSA SORP (Becton Dickinson) and analysed with FCS express flow cytometry software version 5.

Plasma results for the chemokine CXCL9 (Figure 5A), which promotes the differentiation and multiplication of leukocytes, and CXCL10 (Figure 5B), which measured immune responses through leukocyte activation and recruitment, were shown to be significantly increased in stressed mice compared to the control group (p<0.01). Treatment with B. *longum* CECT 30763 significantly reduced (p<0.05) this alteration in both cases. These chemokines are increased in patients with elevated levels of anxiety and depressive symptoms, so treatment with B. *longum* CECT 30763 may improve immune markers associated with depressive and anxiogenic states.

The results of CCL2 (Figure 5C), a chemokine involved in the recruitment of monocytes, memory T cells, and dendritic cells at sites of inflammation, were increased in the stressed mouse group (p<0.001), and administration of B. *longum* CECT 30763 restored this alteration (p<0.001). This chemokine is increased in patients with major depressive disorder, so these results would indicate that B. *longum* CECT 30763 is capable of reducing this inflammatory marker of depression in plasma.

The results of IL-6 (Figure 5D) and TNF-α (Figure 5E) showed a significant increase in the stressed group (p<0.0001 and p<0.001, respectively). Treatment reduced this alteration in both cases, although it was not significant at a value of p<0.05. IL-6 plays a crucial role in the pathogenesis of depression and is one of the most highly increased cytokines in the serum of depressed subjects in both clinical and preclinical trials. Meanwhile, there is a relationship between serum levels of TNF-α and subjects with anxiety disorders. It could therefore be affirmed that B. *longum* CECT 30763 exerts a positive role in the immune response related to depression and anxiety.

Overall, the results indicate that B. *longum* CECT 30763 exerts a positive role as it restores 5 chemokines and cytokines in plasma, which are altered in patients with symptoms of depression and/or anxiety. Some of these inflammatory mediators are also involved in the development of cardiovascular pathologies. Particularly, CCL2 plays a crucial role in the initial development of pathologies of this type, as it promotes the infiltration of macrophages and other immune system cells into cardiac tissue, activating, among other things, a cascade of pro-inflammatory cytokine production, including IL-6 and TNF-α. In this case, *B. longum* CECT 30763 may play an important role in cardiovascular health, as the reduction of these chemokines has been linked to a decrease in interstitial fibrosis and lower cardiac dysfunction in cardiovascular disease.

The results of cytometry in spleen cells and small intestine cells (Figures 6A and 6B, respectively), showed that regulatory T cells (Tregs) were significantly decreased as a result of chronic stress and that B. *longum* CECT 30763 was able to increase them by exerting an anti-inflammatory effect after repeated exposure to stress. Furthermore, in the model of acute social stress (stress for 5 minutes on a single day) the administration of the bacterium was shown to be able to increase Treg lymphocytes significantly (p<0.001) and independently of the effect of stress (Figure 6C). This latter result suggests that B. *longum* CECT 30763 has *per* se an anti-inflammatory effect which, when exerted at early stages of exposure to stress, reduces the adverse effects of continued exposure.

### EXAMPLE 4. Effect of B. longum strain CECT 30763 on the neuroendocrine response in an animal model of chronic social stress-induced depression.

The results of treatment with *B. longum* CECT 30763 on the endocrine response were measured by analysing in plasma the release of corticosterone, which is the main hormone released into the bloodstream in response to activation of the HPA axis that occurs when the body perceives stress. To prepare the sample, cold methanol was added at a 1:3 ratio to the plasma for deproteinisation, and centrifuged at 13,000 rpm at 4°C for 10 minutes. The supernatant was injected into the mass spectrophotometer.

The results (Figure 7A) showed that stress for 10 consecutive days is capable of significantly increasing (p<0.0001) plasma levels of corticosterone. Treatment with B. *longum* CECT 30763 was able to slightly reduce levels of corticosterone, although this reduction was not significant.

The dopamine response was evaluated because dopamine plays an important role in stress-induced depression. Specifically, plasma dopamine (DA) was measured. The same method used for corticosterone was used to measure dopamine.

The results (Figure 7B) showed that the stressed group of mice showed a clear, though not significant increase in levels of plasma DA and that treatment with B. *longum* CECT 30763 was capable of significant normalisation (p<0.01, Figure 7B).

The expression of two major DA receptors was analysed in the prefrontal cortex of the brain, which is affected both in animal models and in patients with depression. The analysis was done by qPCR, and to that end, the RNA was extracted with trizole, the cDNA was obtained by reverse transcription and qPCR was performed with gene-specific primers.

The primers used to identify the receptors were as follows: D2L: 5'-3'F-GGAGTTTCCCAGTGAACAGGCGG (SEQ ID NO: 7) and 5'-3'R-TTGCTATGTAGACCGTGGTGGGATG.(SEQ ID NO: 8)
D1: 5'-3'F-CAGGTAAACCAGATTACAGTCCTTG (SEQ ID NO: 9) and 5'-3'R-TTAGGATGCTATAGACTCTGCCCTA (SEQ ID NO: 10).

The reference or "housekeeping" gene against which the gene expression of both receptors was compared and quantified was RPL19 and the primers used were: 5'-3'F-CCTTGTCTGCCTTCAGCTTGT (SEQ ID NO: 11) and 5'-3'R-GAAGGTCAAAGGGAATGTGTTCA (SEQ ID NO: 12).

The results showed that the D2L receptor (Figure 8A) is significantly decreased (p<0.05) in the stressed mice group. Treatment with the bacterium restored this alteration (p<0.05). As for the D1R receptor (Figure 8B), a non-significant decrease in receptor expression was observed and treatment shows a trend in restoring this slight alteration (Figure 8B).

These results, overall, show that B. *longum* CECT 30763 is capable of improving the endocrine response as well as the dopamine response in an animal model of chronic social stress-induced depression.

### EXAMPLE 5. Effect of the B. longum strain on the liver response in an animal model of chronic social stress-induced depression.

The expression of genes encoding transforming growth factor beta 1 (TGFβ1), Type 1 collagen alpha 1 chain (Col1a1) and type 3 collagen alpha 1 chain (Col3a1) (Figures 9A, B, C, respectively) was analysed in the liver of mice in the model of social stress by RT-qPCR.

After analysing the data, it was observed that type I collagen is significantly increased in the chronic stress group and that probiotic treatment was capable of preventing this effect. The results for the expression of type III collagen seem to follow a similar trend, although no statistically significant differences were observed between the groups.

However, although an increase in the expression of TGFβ1 was expected, as it is a profibrotic molecule expressed by myofibroblasts to promote collagen production, no changes were observed. This can be explained by the fact that this molecule is part of the first steps of the fibrosis pathway, and, therefore, after 10 days of exposure of the mice to stress, the expression is no longer increased, but the protein or its target genes, such as collagens, are.

The following primers were used:
TGFβ1: 5'-3'F- ACCATGCCAACTTCTGTCTG (SEQ ID NO: 13) and 5'-3'R-CGGGTTGTGTTGGTTGTAGA (SEQ ID NO: 14).
Col1a1: 5'-3'F-AATGGCACGGCTGTCTGCGA (SEQ ID NO: 15) and 5'-3'R-AGCACTCGCCCTCCCGTCTT (SEQ ID NO: 16)
Col3a1: 5'-3'F-CTGTAACATGGAAACTGGGGAAA (SEQ ID NO: 17) and 5'-3'R-CCATAGCTGAACTGAAAACCACC (SEQ ID NO: 18)

In relation to metabolic health, *B. longum* CECT 30763 has been observed to have protective effects on some of the chronic stress-induced changes in the expression of enzymes related to fatty acid metabolism in the liver, which is the main organ responsible for their metabolism. Specifically, the expression of the ACC1 (acetyl-CoA carboxylase 1) enzyme (Figure 10A) decreases with stress. This enzyme is involved in the *de novo* synthesis of fatty acids and the decreased activity has been shown to result an increase in the storage of fat and triglycerides in the blood [Chow, J.D., et al., Genetic inhibition of hepatic acetyl-CoA carboxylase activity increases liver fat and alters global protein acetylation. Mol Metab, 2014. 3(4): p. 419-31.]. Treatment with B. *longum* CECT 30763 is capable of increasing its expression in the liver of mice with chronic stress in a statistically significant manner. The following primers were used:
ACC1: 5'-3'F- TAATGGGCTGCTTCTGTGACTC (SEQ ID NO: 19) and 5'-3'R-CTCAATATCGCCATCAGTCTTG (SEQ ID NO: 20).

Furthermore, stress increases the expression of the DGAT (diacylglycerol O-acyltransferase 1) enzyme (Figure 10B), which is involved in the synthesis of triglycerides in the liver from dietary fatty acids, and reduces the expression of the CPT1a (carnitine palmitoyltransferase 1A) enzyme gene (Figure 10C) involved in the oxidation of fatty acids and their use as an energy source. All this leads to an increase in the accumulation of lipids in the liver and promotes hepatic steatosis . This imbalance between DGAT/CPT1a (Figure 10D) is reversed by *B. longum* CECT 30763, improving metabolism and decreasing the risk of developing related alterations. The following primers were used:
DGAT: 5'-3'F-TCCGTCCAGGGTGGTAGTG (SEQ ID NO: 21) and 5'-3'R-TGAACAAAG AATCTTGCAGACGA (SEQ ID NO: 22).
CPT1a: 5'-3'F-TTTGAATCGGCTCCTAATGG (SEQ ID NO: 23) and 5'-3'R-CCCAAGTATCCACAGGGTCA (SEQ ID NO: 24).

The reference gene used was RPL19, the primers of which have already been specified in Example 4.

### EXAMPLE 6. Effect of B. longum strain CECT 30763 on oxidative stress in an animal model of chronic social stress-induced depression.

The presence of 8-hydroxy-2'-deoxyguanosine (8-OHdG) (Figure 11), a biomarker of DNA damage caused by oxidative stress, has been analysed in cardiac tissue. This biomarker has been linked to cardiac disorders, particularly in hypertensive pathology. To study the presence of DNA damage caused by oxidative stress, immunofluorescence techniques were performed on 10 µm thick cross-sections of the hearts of mice from the different experimental groups. These sections were first incubated with a monoclonal mouse primary antibody against 8-OHdG and then with a secondary antibody against the fluorescent mouse primary antibody (Alexa Fluor^{®} 488). Phalloidin, a probe conjugated with a red fluorescent dye (Alexa Fluor^{®} 546) that specifically labels F-actin fibres, and the fluorescent marker DAPI, which specifically labels cell nuclei, were also used. To quantify this oxidative damage, images obtained under a 16x microscope with a fluorescence lamp were analysed and the percentage of area marked by the 8-OHdG antibody was calculated from the total tissue area.

Data analysis shows that chronic stress in mice induces cellular stress due to neurohormonal stimulation or excessive inflammation that alters the antioxidant balance of cardiomyocytes (heart muscle cells), which increases the generation of reactive oxygen species (ROS), inducing damage to DNA and its expression and, accordingly, harmful effects on cell function. This is reflected in the significant increase (p<0.05) of - hydroxy-2'-deoxyguanosine (8-OHdG) in the heart. Treatment with B. *longum* CECT 30763 decreases DNA damage caused by oxidative stress in heart tissue in the model of chronic stress in a statistically significant manner (p<0.01) (Figure 11), thus avoiding the harmful effects arising therefrom, such as increased cell apoptosis, fibrosis formation or the likelihood of arrhythmias. Therefore, it can be affirmed that B. *longum* CECT 30763 exerts a positive role on cardiac function and may prevent or delay the onset of future cardiovascular diseases associated with the animal model of chronic stress-induced depression.

## Claims

1. A strain of *Bifidobacterium longum* with deposit number CECT 30763.

2. A strain derived from the strain according to claim 1.

3. The strain according to claim 1 or 2, wherein the strain is a genetically modified mutant or a spontaneous mutant.

4. The strain according to any one of claims 1 to 3, wherein said strain is in the form of viable cells or in the form of non-viable cells.

5. A cellular component, metabolite, secreted molecule or any of the combinations thereof, obtained from the strain according to any one of claims 1 to 4.

6. A composition comprising the strain according to any one of claims 1 to 4, or a cellular component, metabolite, secreted molecule or any of the combinations thereof according to claim 5.

7. The composition according to claim 6, wherein the composition further comprises at least one bioactive component.

8. The composition according to claim 6 or 7, wherein the composition further comprises at least one microorganism other than the strain according to any of claims 1 to 4.

9. The composition according to claim 8, wherein the microorganism is an intestinal bacterium or a lactic bacterium.

10. The composition according to any one of claims 6 to 9, wherein said composition is a pharmaceutical composition.

11. The composition according to claim 10, wherein the composition further comprises at least one pharmaceutically acceptable carrier and/or excipient.

12. The composition according to any one of claims 10 or 11, wherein said composition is presented in a form adapted for oral, sublingual, nasal, bronchial, intrathecal, lymphatic, rectal, transdermal, inhaled or parenteral administration.

13. The composition according to any one of claims 6 to 9, wherein said composition is a nutritional composition.

14. The composition according to claim 13, wherein said nutritional composition is a food, a supplement, a nutraceutical, a probiotic or a symbiotic.

15. The composition according to claim 14, wherein said food is selected from the list consisting of a dairy product, a plant product, a meat product, a snack, chocolate, beverage and infant food.

16. The composition according to any one of claims 6 to 15, wherein said composition has a strain concentration of between 10⁴ and 10¹⁴ colony-forming units (cfu) per gram or millilitre of final composition.

17. The strain according to any one of claims 1 to 4, a cellular component, metabolite, secreted molecule or any of the combinations thereof according to claim 5, or the composition according to any one of claims 6 to 16, for use as a medicament.

18. The strain according to any one of claims 1 to 4, a cellular component, metabolite, secreted molecule or any of the combinations thereof according to claim 5, or the composition according to any one of claims 6 to 16, for use in the prevention and/or treatment of at least one mood disorder in a subject.

19. The strain according to any one of claims 1 to 4, a cellular component, metabolite, secreted molecule or any of the combinations thereof according to claim 5, or the composition according to any one of claims 6 to 16, for use as an adjuvant in the treatment of at least one mood disorder in a subject.

20. A strain, cellular component, metabolite, secreted molecule or any of the combinations thereof, or the composition for use according to claim 18 or 19, wherein the mood disorder is selected from the list consisting of: depression, anxiety, major depression, atypical depression, typical or melancholic depression, psychotic depression, catatonic depression, prenatal depression, postpartum depression, bipolar disorder, seasonal affective disorder, stress, dysthymia, depressive personality disorder, double depression, unspecified depressive disorder, recurrent brief depressive disorder, minor depression, mood disorder induced by substance abuse, mood disorder induced by drug use, and any combination thereof.

21. The strain, cellular component, metabolite, secreted molecule or any of the combinations thereof, or the composition for use according to claim 20, wherein the mood disorder is depression and/or anxiety.

22. The strain according to any one of claims 1 to 4, a cellular component, metabolite, secreted molecule or any of the combinations thereof according to claim 5, or the composition according to any one of claims 6 to 16, for use in the prevention and/or treatment of at least one cardiometabolic disorder in a subject.

23. The strain according to any one of claims 1 to 4, a cellular component, metabolite, secreted molecule or any of the combinations thereof according to claim 5, or the composition according to any one of claims 6 to 16, for use as an adjuvant in the treatment of at least one cardiometabolic disorder in a subject.

24. The strain, cellular component, metabolite, secreted molecule or any of the combinations thereof, or the composition for use according to claim 22 or 23, wherein the cardiometabolic disorder is selected from the list consisting of a cardiovascular disease, metabolic syndrome, type 2 diabetes, obesity, hyperlipidaemia, hyperglycaemia, and any combination thereof.

25. The strain according to any one of claims 1 to 4, a cellular component, metabolite, secreted molecule or any of the combinations thereof according to claim 5, or the composition according to any one of claims 6 to 16, for use in the prevention and/or treatment of at least one liver disorder in a subject.

26. The strain according to any one of claims 1 to 4, a cellular component, metabolite, secreted molecule or any of the combinations thereof according to claim 5, or the composition according to any one of claims 6 to 16, for use as an adjuvant in the treatment of at least one liver disorder in a subject.

27. The strain, cellular component, metabolite, secreted molecule or any of the combinations thereof, or the composition for use according to claim 25 or 26, wherein the liver disorder is selected from the list consisting ofhepatic steatosis , liver fibrosis, hepatitis, hepatocellular carcinoma, liver failure and any combination thereof.

28. The strain according to any one of claims 1 to 4, a cellular component, metabolite, secreted molecule or any of the combinations thereof according to claim 5, or the composition according to any one of claims 6 to 16, for use in the prevention and/or treatment of comorbid mood disorders in a subject.

29. The strain according to any one of claims 1 to 4, a cellular component, metabolite, secreted molecule or any of the combinations thereof according to claim 5, or the composition according to any one of claims 6 to 16, for use as an adjuvant in the prevention and/or treatment of comorbid mood disorders in a subject.

30. The strain, cellular component, metabolite, secreted molecule or any of the combinations thereof, or the composition for use according to claim 28 or 29, wherein the mood disorder is selected from the list consisting of: depression, anxiety, major depression, atypical depression, typical or melancholic depression, psychotic depression, catatonic depression, prenatal depression, postpartum depression, bipolar disorder, seasonal affective disorder, stress, dysthymia, depressive personality disorder, double depression, unspecified depressive disorder, recurrent brief depressive disorder, minor depression, mood disorder induced by substance abuse, mood disorder induced by drug use, and any combination thereof.

31. The strain according to any one of claims 1 to 4, a cellular component, metabolite, secreted molecule or any of the combinations thereof according to claim 5, or the composition according to any one of claims 6 to 16, for use in the prevention and/or treatment of cardiometabolic comorbidities in a subject.

32. The strain according to any one of claims 1 to 4, a cellular component, metabolite, secreted molecule or any of the combinations thereof according to claim 5, or the composition according to any one of claims 6 to 16, for use as an adjuvant in the prevention and/or treatment of cardiometabolic comorbidities in a subject.

33. The strain, cellular component, metabolite, secreted molecule or any of the combinations thereof, or the composition for use according to claim 31 or 32, wherein the cardiometabolic comorbidities comprise one or more cardiometabolic disorders selected from the list consisting of: a cardiovascular disease, metabolic syndrome, type 2 diabetes, obesity, hyperlipidaemia, hyperglycaemia, and any combination thereof.

34. The strain according to any one of claims 1 to 4, a cellular component, metabolite, secreted molecule or any of the combinations thereof according to claim 5, or the composition according to any one of claims 6 to 16, for use in the prevention and/or treatment of liver comorbidities in a subject.

35. The strain according to any one of claims 1 to 4, a cellular component, metabolite, secreted molecule or any of the combinations thereof according to claim 5, or the composition according to any one of claims 6 to 16, for use as an adjuvant in the prevention and/or treatment of liver comorbidities in a subject.

36. The strain, cellular component, metabolite, secreted molecule or any of the combinations thereof, or the composition for use according to claim 34 or 35, wherein the liver comorbidities comprise one or more liver disorders selected from the list consisting of hepatic steatosis , liver fibrosis, hepatitis, hepatocellular carcinoma, liver failure and any combination thereof.

37. The strain according to any one of claims 1 to 4, a cellular component, metabolite, secreted molecule or any of the combinations thereof according to claim 5, or the composition according to any one of claims 6 to 16, for use in the prevention and/or treatment of one or more cardiometabolic disorders and one or more liver disorders comorbid with at least one mood disorder in a subject.

38. The strain according to any one of claims 1 to 4, a cellular component, metabolite, secreted molecule or any of the combinations thereof according to claim 5, or the composition according to any one of claims 6 to 16, for use as an adjuvant in the prevention and/or treatment of cardiometabolic and liver disorders comorbid with at least one mood disorder in a subject.

39. Use of the strain according to any one of claims 1 to 4, a cellular component, metabolite, secreted molecule or any of the combinations thereof according to claim 5, or the composition according to any one of claims 6 to 16, for preparing a food.
